# EUROPEAN PATENT SPECIFICATION

(11) **EP 0 874 610 B1**
(45) Date of publication and mention of the grant of the patent: **26.06.2002**
(21) Application number: 96929155.8
(22) Date of filing: 12.09.1996
(51) Int. Cl.: A61F 13/15

(54) **HIGHLY ABSORBENT TRANSFER LAYER STRUCTURE**
HOCHABSORBIERENDER ARTIKEL MIT EINER SCHICHTSTRUKTUR ZUM ABLEITEN VON FLÜSSIGKEIT
STRUCTURE DE COUCHE DE TRANSFERT FORTEMENT ABSORBANTE

(30) Priority: 17.10.1995 CA 2160757
(43) Date of publication of application: 04.11.1998
(73) Proprietor: JOHNSON & JOHNSON INC., Montreal, Quebec H1N 2G4 (CA)
(72) Inventor: BOULANGER, Roger, Sainte-Julie, Quebec J3E 1CO (CA)
(74) Representative: Mercer, Christopher Paul
(86) International application number: CA9600607
(87) International publication number: WO9714384

(56) References cited:
- EP-A- 0 547 498
- WO-A-90/11170
- WO-A-91/01766
- WO-A-92/16681
- WO-A-93/01779
- GB-A- 2 266 465

## Description

The present invention relates to the art of manufacturing structures for handling body exudate. More particularly, the invention relates to a binder consolidated liquid-absorbent article characterized by a non-uniform binder distribution profile that allows the liquid-absorbent article to take-up liquid faster. The liquid-absorbent article is well suited for use as a transfer layer in a sanitary napkin or another disposable sanitary protection product. The invention also relates to a method for manufacturing the novel liquid-absorbent article.

### BACKGROUND OF THE INVENTION

Sanitary napkins are multi-layered structures that normally comprise a body contacting liquid-pervious cover layer, an absorbent system and a barrier layer that prevents fluid entrapped in the absorbent system to egress from the garment facing surface of the sanitary napkin. Usually, the absorbent system is a dual-layer arrangement. It includes an absorbent core whose primary function is to entrap the liquid discharge permanently and a transfer layer whose function is to collect the body exudate quickly and then meter the liquid to the absorbent core. Liquid discharged on such composite absorbent systems will rapidly ingress the transfer layer due to its highly porous network. From the transfer layer, liquid migrates toward the absorbent core by capillary pressure because of the substantial difference in wicking power between the different materials. The liquid migration is well-controlled, occurring at the rate of acceptance. of the absorbent core.

The transfer layer is in the form of a sheet and it is typically made from cellulosic fibers by an air-laid process. To consolidate the porous sheet so that it can withstand the mechanical stresses normally encountered in use, without suffering a loss of structural integrity, binder is applied on both main surfaces of the transfer layer, namely on its liquid-acquisition surface that faces the cover layer and on its liquid-release surface oriented toward the absorbent core. The amount of binder applied to both surfaces is the same.

Conventional wisdom dictates that from the point of view of liquid absorption performance, the presence of binder on the transfer layer is undesirable. The binder sites that link the fibers are thought to behave as local barriers preventing liquid from freely migrating in and within the porous structure. The requirement of fast liquid penetration is particularly important for the liquid-acquisition surface of the transfer layer that should be able to take-up rapidly large amounts of liquids otherwise, the sanitary napkin may fail due to overflow leakage at the transfer layer level.

GB-A-2266465 provides an absorbent article comprising a laminate structure wherein a first layer includes a mixture of resilient fibers and from 5-20% heat actuable binding fibers and a second layer which includes a mixture of absorbent fibers and 5-15% binding fibers.

### OBJECTS AND STATEMENT OF THE INVENTION

An object of the present invention is a binder-consolidated liquid-absorbent article manifesting a short liquid-penetration time.

Another object of the invention is a method for manufacturing the aforementioned liquid-absorbent article.

As embodied and broadly described herein, the invention provides an integrally formed liquid-absorbent article of particulate material including first and second zones in intimate fluid communicative relationship, each zone having a multiplicity of inter-particle interstices admitting passage of liquid, whereby liquid contained in one of said zones can migrate toward the other of said zones, said liquid-absorbent article containing binder, said first zone having a higher average concentration of binder than said second zone.

For the purpose of this specification "particle" means a small unit of material without limitation of shape. A fiber that is characterized by a geometrical extension along a preferential direction will be considered a particle. Therefore, "particle" and "particulate" cover a material made of fibers, particles having non-fibrous identity, such as sphagnum moss, or a combination of both. Also, the term "absorbent" will be used to designate a structure capable of taking-up liquid, without any regard to liquid-retentivity. As such, a material well suited for use as a transfer layer, such as a low density cellulose web, will be considered "absorbent" although the material has poor liquid-retentivity characteristics, i.e., it is designed specifically to allow liquid to easily egress its porous structure so the liquid can transfer itself to the absorbent core. Finally, "binder concentration" means the weight of binder (solids) per unit weight of particulate material and other constituents that may be included with the particulate material but excluding the binder (solids). The "average concentration of binder" for a given zone is obtained by dividing the weight of binder (solids) contained in the zone by the weight of the zone (solids) from which the weight of binder has been subtracted.

Without intent of being bound by a specific theory, the present inventor believes that the non-uniform binder distribution profile creates within the liquid-absorbent article an intermediate zone having a high void volume (low density). Liquid migrating through the porous network of the liquid-absorbent article will have a tendency to spread along the X-Y plane, once it reaches the high void volume zone. The liquid front, now distributed over a larger surface area advances more rapidly since it is channelled through a larger number of capillary passageways. Such liquid-absorbent structure is particularly useful as a transfer layer for a disposable absorbent product, such as a sanitary napkin, diaper, adult incontinence brief, urinary pad, wound dressing and nursing pad. In normal use, the body exudate discharged over the disposable absorbent product is limited to a small impact area. Prior art transfer layers are not designed to allow the advancing liquid front to expand laterally significantly. As a result, the liquid is constrained to flow through a comparatively small number of capillaries. In contrast, the novel liquid-absorbent structure induces the liquid front to flow laterally and also in the Z direction (along the thickness of the liquid-absorbent article). This feature enables the liquid to travel faster.

Surprisingly, the liquid-absorbent article manifests a higher liquid penetration rate when the liquid is deposited on the surface on which the binder concentration is highest. This is contrary to conventional wisdom that dictates that an increase in the amount of binder on the liquid-acquisition surface of a porous structure reduces the rate of penetration of liquid in the structure.

In a preferred embodiment, the liquid-absorbent article according to the invention is made of fibers. Cellulosic fibers are most preferred although other fibers could also be used without departing from the spirit of the invention. The length of the fibers influences the formation of the intermediate high void volume zone. Best results have been obtained with fibers having a length not exceeding 3.5 millimeters (mm).

As embodied and broadly described herein, the invention also provides a transfer layer for a disposable absorbent product comprising the integrally formed liquid-absorbent article as defined above.

As embodied and broadly described herein, the invention also provides a method for manufacturing a liquid-absorbent article as defined above, said method comprising the steps of:
- forming particulate material into a sheet having first and second main faces, said sheet having a multiplicity of inter-particle interstices admitting passage of liquid; and
- applying binder to said sheet to provide a higher concentration of binder in a vicinity of said first main face than a concentration of binder in a vicinity of said second main face.
   In a most preferred embodiment, the liquid-absorbent article is manufactured from cellulosic fibers by the well-known air-laid process. Different quantities of binder are deposited on the opposite main surfaces of the cellulosic sheet to provide a non-uniform binder distribution profile. The liquid-absorbent article is then processed according to known techniques to incorporate it in a disposable absorbent product, such as a sanitary napkin.

### BRIEF DESCRIPTION OF THE DRAWINGS

- Figure 1 is an enlarged cross-sectional view of a liquid-absorbent article according to the present invention;
- Figure 2 is a graph illustrating the binder concentration in the liquid-absorbent article shown in Figure 1; and
- Figure 3 is a cross-sectional view of a sanitary napkin with the liquid-absorbent article used as transfer layer.

### DESCRIPTION OF A PREFERRED EMBODIMENT

The present invention provides an integrally formed liquid-absorbent article suitable for use as a transfer layer in a sanitary absorbent product, such as a sanitary napkin. The liquid absorbent article is characterized by a non-uniform binder deposition profile. In an exemplary embodiment, the transfer layer is manufactured in a sheet form from cellulosic fibers according to the well-known air-laid process. The resulting sheet is subjected to binder treatment that consists of spraying one main face of the sheet with binder, letting the binder set and then spraying the other face of the sheet with binder. The amount of binder deposited at each spraying step is different to create a higher binder concentration near one surface of the liquid-absorbent article than near the other surface.

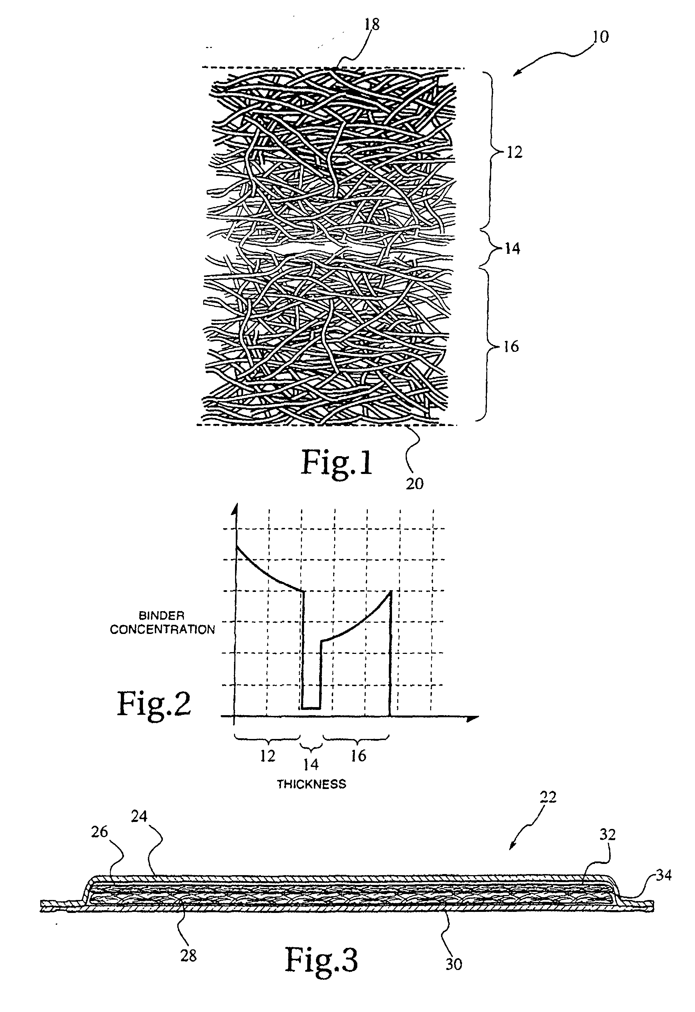

Figure 1 provides an enlarged cross-sectional view of the liquid-absorbent article designated comprehensively with the reference numeral 10. The liquid-absorbent article 10 includes three superposed layers, namely an upper layer 12, an intermediate layer 14 and a lower layer 16. The upper layer 12 and the lower layer 16 have approximately the same thickness, while the intermediate layer 14 is much thinner. The upper layer 12 is distinguished from the two other layers by a comparatively high binder content. The binder concentration profile is at a maximum level at the upper surface 18, where the binder has been deposited in liquid form by spraying. As depth increases, the binder concentration progressively diminishes toward the interface between the upper layer 12 and the intermediate layer 14, where virtually no binder exists. This negative binder concentration gradient is the result of a migration of the liquid binder substance that occurs immediately after the binder has been sprayed. The liquid binder sinks within the porous structure due to capillary pressure and gravity. However, the rate of progression of the liquid front gradually diminishes because the viscosity of the liquid increases as the curing process nears completion. The migration process is completed when the binder is fully cured.

The structure of the lower layer 16 is similar to the upper layer 12 except that it contains much less binder. The binder concentration of the lower layer 16 peaks at the lower surface 20 and gradually decreases in the upward direction until the interface lower layer 16/intermediate layer 14 where it drops to negligible levels.

The intermediate layer 14 is formed as a result of the presence of the binder consolidated upper and lower layers 12 and 16. Without intent of being bound by a particular theory it is believed that immediately after the binder is cured the fibers in the upper layer 12 and in the lower layer 16 are linked to one another and thus fixed in predetermined spacial positions, except the fibers at the intervening region between these two layers where the amount of binder is minimal. During subsequent manipulations and handling of the liquid-absorbent article, the layers 12 and 16 may slightly separate from one another creating between them the intermediate layer 14. The intermediate layer 14 is characterized by a much lower density (higher void volume) than layers 12 and 14 and it can transport liquid much faster than layers 12 and 14. The graph of Figure 2 illustrates the binder deposition profile of the liquid-absorbent article 10.

When liquid is discharged on the liquid-absorbent article 10, say the surface 18, the liquid begins penetrating the porous network along a vector that is generally transverse to the plane of the liquid-absorbent article, i.e., the liquid front advances vertically and there is very little lateral dispersion. However, when the liquid reaches the intermediate layer 14, it spreads apart considerably and enters the lower layer 16 distributed over a larger surface area. The liquid travels faster through lower layer 16 because it is channelled through an increased number of capillary passageways.

In a specific example, the liquid-absorbent article 10 is made from cellulosic fibers (100% Southern Pine Kraft) by an air-laid process. The cellulosic web prior to the binder deposition has a basis weight of 66.2 grams per meter squared (g/m²). The total amount of binder deposited on the cellulosic web ranges from about 10% to about 25% by weight of solids in the liquid-absorbent article (cellulosic fibers plus binder). In a preferred embodiment the amount of binder deposited on the cellulosic web is 13.8 g/m² of solids. EVA (ethylene vinyl acetate) binder has been found satisfactory. Acrylic binder can also be used. No less than 75% of the total amount of binder applied to the web is deposited on one surface of the web, while the balance (25% maximum) on the other surface. Most preferably, 85% of the total amount of binder (11.73 g/m² of solids in the example given above) is applied to one side of the web while the other side receives 15% (2.07 g/m²). To effect the non-uniform binder deposition, the concentration of the liquid binder is changed, not the amount of material sprayed. Thus, both sides of the web receive the same amount of liquid, but the concentration of binder in the liquid varies. Other methods to effect the non-uniform binder deposition can also be used. For example, it could be considered to spray or otherwise deposit liquid binder having the same concentration on both faces of the web, except that one face receives more material than the other face.

The properties of the liquid-absorbent article 10 constructed under the specific example discussed above are compared with a control sample in a test. The control sample is identical to the invention except that the binder is applied uniformly on both surfaces of the cellulosic web. Thus each side receives 6.9 g/m² of binder (solids). The purpose of the test is to determine the capillary attraction developed by an absorbent material on a fluid.

The instrument disclosed in the US patent 5,361,627 in the name of Johnson & Johnson Inc. issued on November 8, 1994 is used for the test procedure. A sample of the material to be tested in the form of a rectangle 5 cm by 5 cm is laid on the sphagnum absorbent core taken from a sanitary napkin available in Canada under the brand name Stayfree Prima. The absorbent core acts as a source of capillary pressure to wick away liquid delivered on the sample. Three (3) cc of test fluid is deposited on the virgin sample and the sensor of the instrument is placed in contact with the absorbing surface of the sample. After 30 minutes from the fluid discharge the pressure reading in millimeters of mercury (mmHg) is recorded. Test fluid is synthetic menstrual liquid without protein having a viscosity of 5.5 cps. The test results are reported in the following table:

| **SAMPLE** | **CAPILLARY PRESSURE (mmHg)** |
|---|---|
| Control | 26.2 |
| Invention (sensor of the instrument in contract with side having the 85% binder portion) | 31.8 |
| Invention (sensor of the instrument in contact with side having the 15% binder portion) | 23.5 |

It will be apparent that the capillary pressure, which is a measure of how well the sample transfers liquid to the core of sphagnum moss material, is higher in the case of the invention (side having 85% binder) than the sample. Also note that the liquid-absorbent article according to the invention performs better when the liquid is deposited on the side containing 85% binder than the side containing 15% binder. These results are very surprising and contrary to conventional wisdom that dictates that high binder concentrations reduce the ability of a porous structure to channel liquid.

The liquid-absorbent article 10 is well-suited for use as a transfer layer in a sanitary napkin. An example of such sanitary napkin is shown in Figure 3. The napkin, comprehensively designated by the reference numeral 22, comprises a liquid-permeable cover layer 24 overlaying a transfer layer 26 manufactured in accordance with the invention. Below the transfer layer 26 is provided an absorbent core 28 that preferably includes sphagnum moss material and it is comparatively thin, i.e., having a few millimeters in thickness. Underneath the absorbent core 28 is provided a barrier layer 30 made of liquid-impervious material, such as polyethylene, to prevent liquid entrapped in the absorbent core 30 from egressing the sanitary napkin 22 and staining the wearer's underpants. The cover layer 24 and the barrier layer 30 are joined along their marginal portions so as to form an enclosure that maintains the absorbent core 28 captive. The joint may be made by means of adhesive or heat-bond.

The transfer layer 26 comprises an upper liquid-acquisition surface 32 through which liquid is received from the cover layer 24. The lower surface 34 of the transfer layer that faces the absorbent core 28 is the liquid-release surface through which liquid is passed to the absorbent core. It is preferred to orient the transfer layer 32 in such a way as to place the side containing the most binder up so it forms the liquid-acquisition surface. As discussed earlier, this configuration provides better performance.

Applications of the product and methods of the present invention for sanitary and other health-care uses can be accomplished by any sanitary protection, incontinence, medical and absorbent methods and techniques as are presently or prospectively known to those skilled in the art. Thus, it is intended that the present application covers the modifications and variations of this invention provided that they come within the scope of the appended claims.

## Claims

1. An integrally formed liquid-absorbent article (10) of particulate material including first (12) and second (16) zones in intimate fluid communicative relationship, and first (18) and second (20) opposite main faces, said first zone (12) including said first main face and said second zone (16) including said second main face, each zone having a multiplicity of inter-particle interstices admitting passage of liquid, whereby liquid contained in one of said zones is capable of migrating toward the other of said zones, said liquid-absorbent article (10) containing binder,
said first zone (12) having a higher average concentration of binder than said second zone (16) **characterised in that**: said first zone (12) has a binder concentration gradient wherein there is a maximum binder concentration at the first face (18) and the binder concentration progressively diminishes towards the interface between the first zone and the second zone, and said second zone has a binder concentration gradient wherein there is a maximum binder concentration at the second face (20) and the binder concentration gradually decreases towards the interface between the first and second zones.

2. An integrally formed liquid-absorbent article (10) as defined in claim 1, wherein said liquid-absorbent article is in the form of a sheet.

3. An integrally formed liquid-absorbent article (10) as defined in claim 2, further including a third zone (14) located between said first zone (12) and said second zone (16), said third zone (14) having a lower density than said first and second zones.

4. An integrally formed liquid-absorbent article (10) as defined in claim 3, wherein said third zone (14) has a lower concentration of binder than said first zone (12) and second zone (16).

5. An integrally formed liquid-absorbent article (10) as defined in claim 1, wherein said liquid-absorbent article includes fibrous material.

6. An integrally formed liquid-absorbent article (10) as defined in claim 5, wherein said fibrous material is cellulose.

7. An integrally formed liquid-absorbent article (10) as defined in claim 1, wherein said liquid-absorbent article includes binder in the range from about 10% to about 25% by weight of solids in said liquid-absorbent article.

8. An integrally formed liquid-absorbent article (10) as defined in claim 7, wherein said binder is selected in the group consisting of EVA and acrylic.

9. An integrally formed liquid-absorbent article (10) as defined in claim 7, wherein said first zone (12) contains no less than about 75% of a total binder content of said liquid-absorbent article.

10. A transfer layer for a disposable absorbent product comprising the integrally formed liquid-absorbent article (10) of any one of claims 1 to 9.

11. A disposable absorbent product including the transfer layer of claim 10.

12. A disposable absorbent product, as defined in claim 11, wherein said disposable absorbent product is selected from the group consisting of sanitary napkin, diaper, adult incontinence brief, urinary pad, wound dressing and nursing pad.

13. A method for manufacturing a liquid-absorbent article according to any one of claims 1 to 9, said method comprising the steps of:
- forming particulate material into a sheet having first and second main faces, said sheet having a multiplicity of inter-particle interstices admitting passage of liquid; and
- applying binder to said sheet to provide a higher concentration of binder in a vicinity of said first main face than a concentration of binder in a vicinity of said second main face.

14. A method as defined in claim 13, wherein said particulate material is formed into a sheet by air laying.

15. A method as defined in claim 13, wherein binder is sprayed on said first and on said second main faces.

16. A method as defined in claim 13, comprising the step of applying binder to said sheet in a range from about 10% to about 25% by weight of solids in said sheet.

## Patentansprüche

1. Integral gebildeter flüssigkeitsabsorbierender Artikel (10) aus einem partikulären Material mit einer ersten Zone (12) und einer zweiten Zone (16), die in intensiver fluidkommunizierender Beziehung stehen, und einer ersten Hauptoberfläche (18) und einer entgegengesetzten zweiten Hauptoberfläche(20), wobei die erste Zone (12) die erste Hauptoberfläche und die zweite Zone (16) die zweite Hauptoberfläche umfaßt, wobei jede Zone mehrere Zwischenteilchenzwischenräume aufweist, die einen Durchgang von Flüssigkeit zulassen, wodurch eine in einer der Zonen enthaltene Flüssigkeit in der Lage ist, zu der anderen der Zonen zu wandern, wobei der flüssigkeitsabsorbierende Artikel (10) ein Bindemittel aufweist, und wobei die erste Zone (12) eine höhere Durchschnittskonzentration des Bindemittels als die zweite Zone (16) aufweist, **dadurch gekennzeichnet, daß** die erste Zone (12) einen Bindemittelkonzentrationsgradienten aufweist, wobei die Bindemittelkonzentration an der ersten Oberfläche (18) maximal ist und nach und nach in Richtung der Grenzfläche zwischen der ersten Zone und der zweiten Zone abnimmt und die zweite Zone einen Bindemittelkonzentrationsgradienten aufweist, und wobei eine maximale Bindemittelkonzentration an der zweiten Oberfläche (20) gebildet ist und die Bindemittelkonzentration allmählich in Richtung der Grenzfläche zwischen der ersten und der zweiten Zone abnimmt.

2. Integral gebildeter flüssigkeitsabsorbierender Artikel (10) nach Anspruch 1, **dadurch gekennzeichnet, daß** der flüssigkeitsabsorbierende Artikel die Form eines Blattes aufweist.

3. Integral gebildeter flüssigkeitsabsorbierender Artikel (10) nach Anspruch 2, **gekennzeichnet durch** eine dritte Zone (14), welche zwischen der ersten Zone (12) und der zweiten Zone (16) angeordnet ist, wobei die dritte Zone (14) eine geringere Dichte als die erste und die zweite Zone aufweist.

4. Integral gebildeter flüssigkeitsabsorbierender Artikel (10) nach Anspruch 3, **dadurch gekennzeichnet, daß** die dritte Zone (14) eine geringere Bindemittelkonzentration als die erste Zone (12) und die zweite Zone (16) aufweist.

5. Integral gebildeter flüssigkeitsabsorbierender Artikel (10) nach Anspruch 1, **dadurch gekennzeichnet, daß** der flüssigkeitsabsorbierende Artikel faserstoffartiges Material umfaßt.

6. Integral gebildeter flüssigkeitsabsorbierender Artikel (10) nach Anspruch 5, **dadurch gekennzeichnet, daß** das faserstoffartige Material Zellulose ist.

7. Integral gebildeter flüssigkeitsabsorbierender Artikel (10) nach Anspruch 1, **dadurch gekennzeichnet, daß** der flüssigkeitsabsorbierende Artikel Bindemittel im Bereich von etwa 10 % bis etwa 25 % des Trockengewichts des flüssigkeitsabsorbierenden Artikels umfaßt.

8. Integral gebildeter flüssigkeitsabsorbierender Artikel (10) nach Anspruch 7, **dadurch gekennzeichnet, daß** das Bindemittel aus Ethylen-Venyl-Acetat (EVA) oder Acryl ist.

9. Integral gebildeter flüssigkeitsabsorbierender Artikel (10) nach Anspruch 7, **dadurch gekennzeichnet, daß** die erste Zone (12) nicht weniger als etwa 75 % des gesamten Bindemittelgehalts des flüssigkeitsabsorbierenden Artikels umfaßt.

10. Weiterleitlage für ein absorbierendes Einwegprodukt mit dem integral gebildeten flüssigkeitsabsorbierenden Artikel (10) nach einem der Ansprüche 1 bis 9.

11. Absorbierendes Einwegprodukt mit der Weiterleitungslage nach Anspruch 10.

12. Absorbierendes Einwegprodukt nach Anspruch 11, **dadurch gekennzeichnet, daß** das absorbierende Einwegprodukt eine Monatsbinde, eine Windel, ein Erwachseneninkontinenzslip, eine Urinvorlage, ein Wundverband oder eine Stilleinlage ist.

13. Verfahren zur Herstellung eines flüssigkeitsabsorbierenden Artikels nach einem der Ansprüche 1 bis 9, wobei das Verfahren die folgenden Schritte umfaßt:
- Formen eines partikulären Material zu einem Blatt mit einer ersten Hauptoberfläche und einer zweiten Hauptoberfläche, wobei das Blatt mehrere Zwischenteilzwischenräume aufweist, die einen Durchgang von Flüssigkeit zulassen; und
- Aufbringen eines Bindemittels auf das Blatt, um in der Nähe der ersten Hauptoberfläche eine höhere Konzentration des Bindemittels als in der Nähe der zweiten Hauptoberfläche zu schaffen.

14. Verfahren nach Anspruch 13, **dadurch gekennzeichnet, daß** das partikuläre Material mittels Luftschichtens in ein Blatt geformt wird.

15. Verfahren nach Anspruch 13, **dadurch gekennzeichnet, daß** das Bindemittel auf die erste Hauptoberfläche und die zweite Hauptoberfläche gesprüht wird,

16. Verfahren nach Anspruch 13, **dadurch gekennzeichnet, daß** das Bindemittel auf das Blatt in einem Bereich von etwa 10 % bis etwa 25 % des Trockengewichts des Blattes aufgetragen wird.

## Revendications

1. Article absorbant les liquides formé d'un seul tenant (10) de matière particulaire incluant des première (12) et deuxième (16) zones en relation intime de communication de fluide, et des première (18) et deuxième (20) faces principales opposées, ladite première zone (12) incluant ladite première face principale et ladite deuxième zone (16) incluant ladite deuxième face principale, chaque zone ayant une multiplicité d'interstices inter-particulaires admettant un passage de liquide, ainsi le liquide contenu dans une desdites zones est capable de migrer vers l'autre desdites zones, ledit article absorbant les liquides (10) contenant un liant, ladite première zone (12) ayant une concentration moyenne en liant supérieure à celle de ladite deuxième zone (16) **caractérisée en ce que** :
ladite première zone (12) possède un gradient de concentration en liant dans lequel il existe une concentration maximale en liant au niveau de la première face (18) et la concentration en liant diminue progressivement vers l'interface comprise entre la première zone et la deuxième zone, et ladite deuxième zone possède un gradient de concentration en liant dans lequel il existe une concentration maximale en liant au niveau de la deuxième face (20) et la concentration en liant diminue progressivement vers l'interface comprise entre les première et deuxième zones.

2. Article absorbant les liquides formé d'un seul tenant (10) tel que défini selon la revendication 1, dans lequel ledit article absorbant les liquides est sous forme d'une feuille.

3. Article absorbant les liquides formé d'un seul tenant (10) tel que défini selon la revendication 2, incluant de plus une troisième zone (14) située entre ladite première zone (12) et ladite deuxième zone (16), ladite troisième zone (14) ayant une densité inférieure à celles desdites première et deuxième zones.

4. Article absorbant les liquides formé d'un seul tenant (10) tel que défini selon la revendication 3, dans lequel ladite troisième zone (14) possède une concentration en liant inférieure à celles de ladite première zone (12) et de ladite deuxième zone (16).

5. Article absorbant les liquides formé d'un seul tenant (10) tel que défini selon la revendication 1, dans lequel ledit article absorbant les liquides inclut une matière fibreuse.

6. Article absorbant les liquides formé d'un seul tenant (10) tel que défini selon la revendication 5, dans lequel ladite matière fibreuse est la cellulose.

7. Article absorbant les liquides formé d'un seul tenant (10) tel que défini selon la revendication 1, dans lequel ledit article absorbant les liquides inclut un liant dans la gamme d'environ 10% à environ 25% en poids des solides dans ledit article absorbant les liquides.

8. Article absorbant les liquides formé d'un seul tenant (10) tel que défini selon la revendication 7, dans lequel ledit liant est choisi dans le groupe formé par EVA et l'acrylique.

9. Article absorbant les liquides formé d'un seul tenant (10) tel que défini selon la revendication 7, dans lequel ladite première zone (12) ne contient pas moins d'environ 75% d'une teneur totale de liant dudit article absorbant les liquides.

10. Couche de transfert pour un produit absorbant jetable comprenant l'article absorbant les liquides formé d'un seul tenant (10) selon l'une quelconque des revendications 1 à 9.

11. Produit absorbant jetable incluant la couche de transfert selon la revendication 10.

12. Produit absorbant jetable tel que défini selon la revendication 11, dans lequel ledit produit absorbant jetable est choisi dans le groupe formé par les serviettes hygiéniques, les couches, les slips d'incontinence pour adulte, les protection urinaires, les pansements pour plaies et les compresses mammaires.

13. Procédé pour fabriquer un article absorbant les liquides selon l'une quelconque des revendications 1 à 9, ledit procédé comprenant les étapes consistant à :
- former une matière particulaire dans une feuille ayant des première et deuxième faces principales, ladite feuille ayant une multiplicité d'interstices inter-particules admettant un passage de liquide ; et
- appliquer un liant à ladite feuille pour fournir une concentration en liant à proximité de ladite première face principale supérieure à une concentration en liant à proximité de ladite deuxième face principale.

14. Procédé tel que défini selon la revendication 13, dans lequel ladite matière particulaire est formée en une feuille par dépôt pneumatique.

15. Procédé selon la revendication 13, dans lequel le liant est pulvérisé sur lesdites première et deuxième faces principales.

16. Procédé selon la revendication 13, comprenant l'étape consistant à appliquer un liant à ladite feuille dans une gamme d'environ 10% à environ 25% en poids de solides dans ladite feuille.
